# EUROPÄISCHE PATENTSCHRIFT

(11) **EP 0 638 098 B1**
(45) Veröffentlichungstag und Bekanntmachung des Hinweises auf die Patenterteilung: **26.06.1996**
(21) Anmeldenummer: 93911483.1
(22) Anmeldetag: 20.04.1993
(51) Int. Cl.: C08F 220/04, C08F 222/00, A61K 7/48

(54) **COPOLYMERISATE AUS CARBONSÄUREN UND QUARTÄREN AMMONIUMVERBINDUNGEN UND IHRE VERWENDUNG ALS VERDICKUNGS- ODER DISPERGIERMITTEL**
COPOLYMERS OF CARBOXYLIC ACIDS AND QUATERNARY AMMONIUM COMPOUNDS AND THEIR USE AS THICKENING OR DISPERSING AGENTS
COPOLYMERISATS D'ACIDES CARBOXYLIQUES ET DE COMPOSES D'AMMONIUM QUATERNAIRE ET LEUR APPLICATION COMME EPAISSISSANTS OU COMME DISPERSANTS

(30) Priorität: 29.04.1992 DE 4213971
(43) Veröffentlichungstag der Anmeldung: 15.02.1995
(73) Patentinhaber: BASF Aktiengesellschaft, D-67063 Ludwigshafen (DE)
(72) Erfinder: SCHADE, Christian, D-6700 Ludwigshafen (DE); SANNER, Axel, D-6710 Frankenthal (DE); WEKEL, Hans-Ulrich, D-6701 Ellerstadt (DE); FROSCH, Franz, D-6702 Bad Duerkheim (DE); WESTENFELDER, Horst, D-6730 Neustadt (DE)
(86) Internationale Anmeldenummer: EP9300952
(87) Internationale Veröffentlichungsnummer: WO9322358

(56) Entgegenhaltungen:
- EP-A- 0 335 624

## Beschreibung

Die vorliegende Erfindung betrifft neue Copoylmerisate aus Carbonsäuren, quartären Ammoniumverbindungen und gegebenenfalls Acrylaten oder Methacrylaten, weiteren copolymerisierbaren Monomeren und Vernetzern. Weiterhin betrifft die vorliegende Erfindung die Verwendung dieser Copolymerisate als Verdickungs- oder Dispergiermittel, insbesondere in kosmetischen Zubereitungen sowie diese Copolymerisate enthaltende kosmetische Zubereitungen.

Als übliche Verdickungsmittel oder Viskositätsregler werden Copolymerisate aus olefinisch ungesättigten Carbonsäuren wie (Meth)Acrylsäure, Maleinsäure oder Maleinsäureanhydrid und hydrophoben Comonomeren wie Estern der (Meth)Acrylsäure sowie gegebenenfalls geringen Mengen eines Vernetzers eingesetzt. Derartige Copolymerisate sind beispielsweise in der EP-A 328 725 (1) und der EP-A 435 066 (2) beschrieben. In Wasser-Öl-Gemischen können solche Polymeren gelegentlich als emulgierende Komponente eingesetzt werden. Die Polymeren erzielen ihre Verdickerwirkung, nachdem ein erheblicher Teil der Säurefunktionen mit einer geeigneten Base neutralisiert wurde; sie liegen dann als Polyanion vor.

Derartige Polymere weisen einige Nachteile auf. Da das hydrophobe Comonomer allgemein in Wasser unlöslich ist, müssen die Polymere häufig in einem organischen Lösungsmittel synthetisiert werden. Derartige Lösungsmittel sind oft gesundheitsgefährdend oder gar toxisch. Durch den Gehalt an hydrophoben Comonomer sind diese Polymere allgemein nur schwer in Wasser dispergierbar. Des weiteren müssen gelegentlich sehr große Mengen des hydrophoben Comonomeren eingesetzt werden. Ein weiterer Nachteil ist die oft geringe Stabilität gegenüber Elektrolyten.

Polymere, die eine große Zahl kationischer Gruppen tragen, sollten ebenfalls als Verdicker oder Dispergierhilfen eingesetzt werden können. Derartige Polymere zeichnen sich durch eine hohe Affinität zu den dispergierten Substanzen aus, deren Oberflächen meist negativ geladen sind. Kationische Polymere können daher oft den gegenteiligen Effekt bewirken und zur Koaleszenz bestehender Dispersionen führen; sie werden deshalb auch bevorzugt als Flockungsmittel eingesetzt.

In der DE-AS 11 08 436 (3) werden Mischpolymerisate aus in Wasser schwerlöslichen, ethylenisch ungesättigten Verbindungen, z.B. Estern ungesättigter Carbonsäuren, und N- oder C-vinylsubstituierten aromatischen Verbindungen, die ein quartäres N-Atom enthalten, z.B. N-Vinyl-N'-benzylimidazolium-chlorid, beschrieben. Die Substanzen werden für die Veredlung von Textilien und zur Herstellung von Filmen und Überzügen empfohlen.

Aufgabe der vorliegenden Erfindung war es daher, neue Polymerisate als Verdickungs- ur.d Dispergiermitte , speziell für kosmetische Zubereitungen, bereitzustellen, welche die Nachteile der Mittel des Standes der Technik nicht mehr aufweisen.

Demgemäß wurde ein Copoylmerisat gefunden, welches erhältlich ist durch radikalisch initiierte Polymerisation von
A) 50 bis 99,99 Gew.-% einer olefinisch ungesättigten C₃- bis C₅-Monocarbonsäure, einer olefinisch ungesättigten C₄- bis C₈-Dicarbonsäure oder ihres Anhydrids oder einer Mischung solcher Carbonsäuren oder Carbonsäureanhydride mit
B) 0,01 bis 50 Gew.-% einer olefinisch ungesättigten quartären Ammoniumverbindung der allgemeinen Formel I oder II 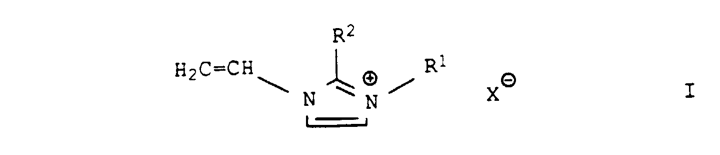 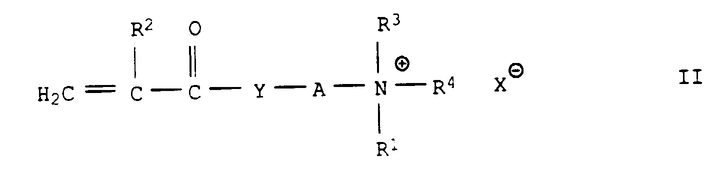 in denen
   - R¹: C₆- bis C₂₀-Alkyl, C₆- bis C₂₀-Alkenyl, C₅- bis C8-Cycloalkyl, Phenyl, Phenyl(C₁- bis C₁₂-alkyl) oder (C₁- bis C₁₂-Alkyl)phenyl bedeutet,
   - R²: Wasserstoff, Methyl oder Phenyl bezeichnet,
   - R³ und R⁴: für Wasserstoff oder C₁- bis C₄-Alkyl stehen,
   - X: Halogen, C₁- bis C₄-Alkylsulfat oder C₁- bis C₄-Alkylsulfonat bedeutet, wobei eine solche C₁- bis C₄-Alkylsulfonat-Gruppe auch als Rest R³ oder R⁴ unter Ausbildung einer Betainstruktur auftreten kann,
   - Y: für O oder NH steht und
   - A: C₁- bis C₆-Alkylen bezeichnet,
   oder einer Mischung solcher Ammoniumverbindungen,
C) 0 bis 49,99 Gew.-% eines Acrylats oder Methacrylats der allgemeinen Formel III 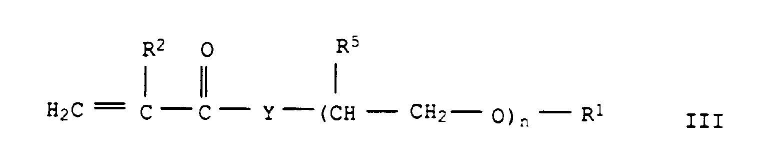 in der R¹, R² und Y die oben genannten Bedeutungen haben, R⁵ Wasserstoff, Methyl oder Ethyl bezeichnet und n für eine Zahl von 0 bis 25 steht,
D) 0 bis 49,99 Gew.-% weiterer copolymerisierbarer Monomerer und
E) 0 bis 5 Gew.-% einer oder mehrerer Verbindungen mit mindestens zwei olefinisch ungesättigten Gruppen im Molekül als Vernetzer.

In einer bevorzugten Ausführungsform ist das erfindungsgemäße Copolymerisat aufgebaut aus
A) 70 bis 99,85 Gew.-% der Carbonsäure-Komponente A,
B) 0,1 bis 29,95 Gew.-% der quartären Ammoniumverbindung I oder II,
C) 0 bis 29,85 Gew.-% des Acrylats oder Methacrylats III,
D) 0 bis 29,85 Gew.-% weiterer copolymerisierbarer Monomerer und
E) 0,05 bis 2 Gew.-% der Vernetzer-Komponente E.

Als Komponente A eignen sich vor allem Acrylsäure, Methacrylsäure oder Maleinsäureanhydrid, daneben aber auch Crotonsäure, 2-Pentensäure, Maleinsäure, Fumarsäure oder Itaconsäure.

Als Reste R¹ in den quartären Ammoniumverbindungen I oder II der Komponente B kommen C₆- bis C₂₀-Alkyl, insbesondere C₁₂- bis C₁₈-Alkyl, z.B. n-Hexyl, n-Octyl, 2-Ethylhexyl, n-Nonyl, iso-Nonyl, n-Decyl, n-Undecyl, n-Dodecyl, n-Tridecyl, iso-Tridecyl, n-Tetradecyl, n-Pentadecyl, n-Hexadecyl, n-Heptadecyl, n-Octadecyl oder n-Eicosyl, C₆- bis C₂₀-Alkenyl, insbesondere C₁₂- bis C₁₈-Alkenyl, z.B. Oleyl, Linolyl oder Linolenyl, C₅- bis C₈-Cycloalkyl, z.B. Cyclopentyl, Cyclohexyl, Methylcyclohexyl oder Dimethylcyclohexyl, Phenyl, Phenyl(C₁- bis C₁₂-alkyl), insbesondere Phenyl(C₁- bis C₄-alkyl), z.B. 2-Phenylethyl, 3-Phenylpropyl, 4-Phenylbutyl oder insbesondere Benzyl, oder (C₁- bis C₁₂-Alkyl)phenyl, insbesondere (C₁- bis C₉-Alkyl)phenyl, z.B. n-Nonylphenyl, n-Octylphenyl oder o-, m- oder p-Tolyl, in Betracht.

Der Rest R² in den Verbindungen I oder II bezeichnet vorzugsweise Wasserstoff oder Methyl.

Die Reste R³ und R⁴ in Verbindung II stehen vorzugsweise für C₁- bis C₃-Alkyl, d.h. für Methyl, Ethyl, n-Propyl oder iso-Propyl.

Das Anion X bedeutet Halogen, vor allem Chlor oder Brom, daneben aber auch Jod, C₁- bis C₄-Alkylsulfat, insbesondere C₁- bis C₃-Alkylsulfat, vor allem Methylsulfat oder Ethylsulfat, C₁- bis C₄-Alkylsulfonat, insbesondere C₁- bis C₃-Alkylsulfonat, vor allem Methylsulfonat oder Ethylsulfonat, oder eine C₁- bis C₄-Alkylsulfonat-Gruppe, insbesondere C₁- bis C₃-Alkylsulfonat-Gruppe, welche als Rest R³ oder R⁴ unter Ausbildung einer Betainstruktur auftritt, z.B. 3-Sulfopropyl.

Die Alkylenbrücke A bezeichnet vorzugsweise geradkettige oder verzweigte C₂- bis C₄-Brückenglieder, z.B. 1,2-Ethylen, 1,3-Propylen, 1,2-Propylen, 2,3-Butylen oder 1,4-Butylen, daneben aber auch Pentamethylen oder Hexamethylen.

Ganz besonders bevorzugt werden quartäre Ammoniumverbindungen I oder II als Komponente B, bei denen
- R¹: C₁₂- bis C₁₈-Alkyl, C₁₂- bis C₁₈-Alkenyl oder Benzyl bedeutet,
- R²: Wasserstoff, Methyl oder Phenyl bezeichnet,
- R³ und R⁴: für C₁- bis C₄-Alkyl stehen,
- X: Chlor, Brom, Methylsulfat, Ethylsulfat, Methylsulfonat, Ethylsulfonat oder eine C₁- bis C₃-Alkylsulfat-Gruppe, welche als Rest R³ oder R⁴ unter Ausbildung einer Betainstruktur auftritt, bedeutet,
- Y: für O oder NH steht und
- A: C₂- bis C₄-Alkylen bezeichnet.
Als Acrylate oder Methacrylate II für die Komponente C kommen insbesondere Stearylacrylat, Stearylmethacrylat, N-Stearylacrylamid, N-Stearylmethacrylamid, Cetylacrylat, Cetylmethacrylat, Laurylacrylat, Laurylmethacrylat, Myristyl (meth) acrylat, Behenylacrylat, Behenylmethacrylat oder ihre Mischungen in Betracht. Sollen mit Ethylenoxid, Propylenoxid oder Butylenoxid umgesetzte (Meth)acrylsäureester oder -amide III eingesetzt werden, liegt der Alkoxylierungsgrad n vorzugsweise bei 3 bis 25.

Als weitere copolymerisierbare Monomere D eignen sich beispielsweise N-Vinylpyrrolidon, N-Vinylcaprolactam, N-Vinylimidazol, C₁- bis C₆-Alkyl(meth)acrylate, z.B. Methyl (meth) acrylat oder Ethyl(meth)acrylat, oder Aminoalkyl(meth)acrylate oder -amide der allgemeinen Formel IV 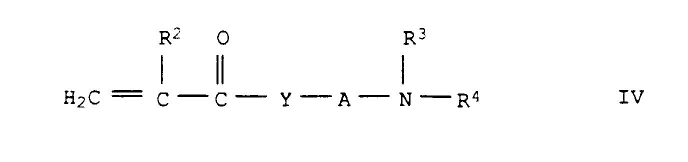 in der die Variablen R², R³, R⁴, Y und A die oben genannten Bedeutungen haben.

Als Vernetzer-Komponente E dient entweder eine wasserlösliche Verbindung wie Divinylethylenharnstoff, Bisacrylamidoessigsäure, Methylenbisacrylamid, Diallylweinsaurediamid oder (Meth)Acrylsäureester von Polyethylenglykolen, beispielsweise Tetraethylenglykoldiacrylat oder eine wasserunlösliche Verbindung wie Ethylenglykoldi(meth)acrylat, Divinylbenzol, Methacrylsäureallylester, Trivinylcyclohexan, ein aliphatisches nichtkonjugiertes Dien und insbesondere ein Allylether des Trimethylolpropans, des Pentaerythrits oder der Saccharose mit mindestens zwei Allylethereinheiten je Molekül. Besonders bevorzugt werden Pentaerythrittriallylether, Oleylmethacrylat, Diallylweinsäurediamid, Bisacrylamidoessigsäure, Methylenbisacrylamid oder (Meth)Acrylsäureester von Polyethylenglykolen.

Die Darstellung der quartären Ammoniumverbindungen I oder II ist im Prinzip bekannt oder kann in Analogie zu bekannten Herstellvorschriften durchgeführt werden. Die Herstellung erfolgt bevorzugt durch Umsetzung eines Amin-funktionalisierten (Meth)Acrylesters oder (Meth)Acrylamids sowie eines N-Vinylimidazolderivats, bevorzugt N-Vinylimidazol, mit beispielsweise einem langkettigen Alkylhalogenid bei höherer Temperatur wahlweise in einem geeigneten Lösungsmittel, das nach Beendigung der Reaktion gewünschtenfalls entfernt wird, oder in Substanz. Bei Bedarf können die Monomeren B beispielsweise durch Umfällen oder Umkristallisieren aus geeigneten Lösungsmittelgemischen gereinigt werden.

Die entsprechenden organischen Halogenide, insbesondere langkettige Alkylchloride, werden mit N-Vinylimidazol oder mit Aminoalkyl(meth)acrylaten oder -amiden bevorzugt in polaren Lösungsmitteln, die bei Raumtemperatur mehr als 0,5 Gew.-% Wasser aufnehmen können, umgesetzt. Beispiele solcher Lösungsmittel sind Alkohole, z.B. Ethanol, n-Butanol, n-Amylalkohol oder Isopropanol, Ketone wie Aceton oder Methylethylketon, Amide, z.B. Dimethylformamid, Dimethylacetamid oder N-Methylpyrrolidon, Nitrile wie Acetonitril, Nitroverbindungen wie Nitromethan oder Nitroethan, Glycolether, z.B. Ethylenglycolmonomethylether oder Diethylenglycoldimethylether, Schwefelverbindungen wie Dimethylsulfoxid oder Sulfolan, Carbonate wie Propylencarbonat oder Diethylencarbonat und Ester wie Ethylacetat. Mischungen dieser Lösungsmittel sind ebenfalls verwendbar. Soweit die entstehenden Produkte in den entsprechenden Medien löslich sind, können auch Wasser oder Mischungen von Wasser mit den beschriebenen Lösungsmitteln verwendet werden. Reaktionstemperaturen oberhalb 40°C sind generell günstig. Zum Erzielen höherer Temperaturen ist in vielen Fällen das Arbeiten unter Drücken bis 30 bar zweckmäßig. Die Reaktion kann zusätzlich durch Zugabe geringer Mengen eines Iod- oder Bromsalzes katalysiert werden.

Die Umsetzung kann in Gegenwart etwa äquimolarer Mengen des Alkylierungsmittels durchgeführt werden. Für die weitere Umsetzung zu Polymeren ist es aber oft nicht notwendig, vollständige Alkylierung zu erzielen. Die Reaktion kann daher auch in Gegenwart unterstöchiometrischer Mengen des Alkylierungsmittels erfolgen. Zur Erzielung höchster Alkylierungsgrade ist es dagegen oft zweckmäßig, die Umsetzung in Gegenwart eines bis zu 4-fachen Überschusses des Alkylierungsmittels durchzuführen. In diesem Fall wird das Produkt oft durch einen Reinigungsschritt vom Überschuß der Alkylierungskomponente befreit.

Die erhaltenen Verbindungen B oder ihre Lösungen können vorteilhaft direkt für die Herstellung der erfindungsgemäßen Copolymerisate eingesetzt werden. Sie können aber auch zunächst gereinigt oder isoliert werden. Dazu können die Verbindungen z.B. in einem geeigneten Lösungsmittel umkristallisiert oder durch ein Fällungsmittel ausgefällt werden. Derartige Solventien sind z.B. Aceton, Ethylacetat, tert.-Butylmethylether oder Kohlenwasserstoffe.

Eine vorteilhafte Möglichkeit zur Darstellung der erfindungsgemäßen Copolymerisate ist die Fällungspolymerisation, bei der die Monomeren, nicht aber das Polymer im eingesetzten Lösungsmittelsystem löslich sind. Geeignete Lösungsmittel sind aromatische wie Toluol oder Xylol oder halogenierte wie 1,1,1-Trichlorethan oder Methylenchlorid sowie semipolare Solventien wie Ketone mit 3 bis 6 C-Atomen oder C₁- bis C₆-Alkylester der Ameisen- und Essigsäure, weiterhin auch unpolare Kohlenwasserstoffe, z.B. Cyclohexan oder Petrolether, sowie Mischungen davon. Das Polymer fällt in Form eines feinteiligen Pulvers an, das abfiltriert und einem geeigneten Trocknungsverfahren unterworfen sowie gewünschtenfalls fein zermahlen wird.

Eine weitere Polymerisationstechnik ist die der umgekehrten Emulsions- oder Suspensionspolymerisation. Im Gegensatz zu Alkyl(meth)acrylaten oder anderen lipophilen Verbindungen lösen sich die beschriebenen kationischen Monomere B zumindest teilweise in Wasser oder Mischungen aus Wasser mit niederen Alkoholen oder Ketonen, so daß die Polymerisation sehr vorteilhaft in der hydrophilen Phase einer Wasser-in-Öl-Emulsion durchgeführt werden kann. Als Öl-Phase wählt man eine unpolare, mit Wasser nicht mischbare Flüssigkeit wie beispielsweise einen Kohlenwasserstoff, speziell Paraffinöl, oder Cyclohexan sowie kosmetische Öle. Je nach der erforderlichen Teilchengröße des Produktes setzt man dem System Schutzkolloide oder Emulgatoren zu.

Zur Initiierung der radikalischen Polymerisation werden geeignete Starter zugesetzt, beispielsweise Alkalimetall- oder Ammoniumpersulfate, Wasserstoffperoxid oder Azostarter oder in der Öl-Phase lösliche Azo- oder Oxo-Starter. Geeignete Initiatorsysteme sind beispielsweise Diacylperoxide wie Dilauroylperoxid, Didecanoylperoxid, Dioctanoylperoxid oder Dibenzoylperoxid, Perester wie z.B. tert.-Butylperneodecanoat, tert.-Butylperethylhexanoat, t-Butylperpivalat, tert.-Amylperneodecanoat, t-Amylperethylhexanoat oder tert.-Butyl-perisobutyrat oder Azoverbindungen wie z.B. 2,2'-Azobis(2,4-dimethylvaleronitril), 2,2'-Azobisisobutyronitril, Dimethyl-2,2'-azobis-isobutyrat oder 2,2'-Azobis(2-methylbutyronitril).

Die erfindungsgemäßen Copolymerisate eignen sich in hervorragender Weise als Verdickungs- oder Dispergiermittel in technischen, pharmazeutischen oder insbesondere in kosmetischen Zubereitungen. Sie können verdickte Gele bilden und Emulsionen dauerhaft stabilisieren, wie es für kosmetische Anwendungen in beispielsweise Cremes, Lotionen oder Gelen erwünscht ist.

Die erfindungsgemäßen Copolymerisate eignen sich allgemein gut zur Verdickung wäßriger Systeme, wie Pigmentanschlämmungen in Wasser, Flüssigwaschmitteln, wäßrigen Polymerlösungen und Polymerdispersionen. Hierzu wird das Polymer durch Zugabe einer Base wie z.B. Triethanolamin, KOH, NaOH, 2-Amino-2-methyl-1-propanol, 2-Amino-2-methyl-1,3-propandiol, Diisopropanolamin oder Tetrahydroxypropylethylendiamin ausreichend neutralisiert. Auf ähnliche Weise lassen sich die Polymere zur Herstellung von verdickten sehr stabilen Emulsionen aus einer Wasser- und einer Ölphase verwenden. Gegenüber herkömmlichen Emulgatoren genügen im allgemeinen geringere Einsatzmengen des Polymers, um dauerhaft stabile Emulsionen zu erhalten.

Gegenstand der vorliegenden Erfindung sind weiterhin kosmetische Zubereitungen, welche die erfindungsgemäßen Copolymerisate als Verdickungs- oder Dispergiermittel in den hierfür üblichen Mengen, also etwa 0,05 bis 2 Gew.-%, enthalten.

Die erfindungsgemäßen Copolymerisate zeichnen sich durch eine Reihe von Vorteilen aus:

Die Polymerisationsreaktion zu ihrer Herstellung kann gewünschtenfalls in Wasser durchgeführt werden, da das einen hydrophoben Rest tragende kationische Monomer B zumindest teilweise wasserlöslich ist.

Dispergierte Phasen tragen zumeist eine negative Partialladung ("triboelektrischer Effekt"). Die kationischen, amphiphilen Gruppen des Polymers besitzten daher eine gute Affinität zu diesen Phasen. Sie können deshalb stabile Emulsionen bei geringer Einsatzmenge bilden.

Durch Einführung kationischer Gruppen in ein - im verdicken-den Zustand - anionisches Polymer nimmt dieses partiell ampholytischen Charakter an. Damit wird die Elektrolytstabili-tät gegenüber herkömmlichen Systemen verbessert.

Kationische Verbindungen besitzen eine hohe Affinität zu Haut und Haar. Die mit den erfindungsgemäßen Copolymerisaten hergestellten Emulsionen eignen sich daher insbesondere für kosmetische Anwendungen im Bereich Haut und Haar.

### Beispiele

Soweit nichts anderes angegeben ist, beziehen sich die Prozentangaben auf das Gewicht.

Herstellung der olefinisch ungesättigten quartären Ammoniumverbindungen B

### Beispiele 1 bis 3

### N-Dodecyl-N'-vinyl-imidazoliumbromid (Beispiel 1)

In einem 2 l-Rührgefäß wurden 98 g N-Vinylimidazol und 258 g Dodecylbromid in 500 ml Ethanol gelöst und bei 50°C 22 h gerührt. Das Rohprodukt wurde eingeengt, in Aceton wieder aufgenommen und durch Zugabe von tert.-Butylmethylether ausgefällt. Das isolierte Produkt wurde in Vakuum getrocknet.

Analog wurden dargestellt:

### Methacryloyloxyethyl-N,N-dimethyl-N-dodecyl-ammoniumchlorid (Beispiel 2)

### N-Dodecyl-N'-vinyl-imidazoliumchlorid (Beispiel 3)

### Beispiele 4 bis 8

### N-Hexadecyl-N'-vinyl-imidazoliumbromid (Beispiel 4)

15 g N-Vinylimidazol und 50 g 1-Bromhexadecan wurden in einem 500 ml-Rührgefäß 8 h bei 60°C gerührt. Zu diesem Ansatz wurden dann unter Rühren 100 ml Essigsäureethylester gegeben. Nach Bildung einer klare- Lösung wurden Heizung und Rührwerk ausgeschaltet. Während des Abkühlens auf Raumtemperatur bildete sich ein farbloser, kristalliner Niederschlag, der abfiltriert und getrocknet wurde.

Analog wurden dargestellt:
Methyacryloyloxyethyl-N,N-dimethyl-N-hexadecyl-ammoniumbromid (Beispiel 5)
N-Octadecyl-N'-vinylimidazoliumchlorid (Beispiel 6)
N-Benzyl-N'-vinyl-imidazoliumchlorid (Beispiel 7)
Methacrylamido-propyl-N,N-dimethyl-N-hexadecylammoniumchlorid (Beispiel 8)
Herstellung der erfindungsgemäßen Copolymerisate
Beispiele 9 bis 26 (Fällungspolymerisate)
In einem 3 l-Planschiffkolben wurden 1000 ml Solvens, 200 g Acrylsäure, die Comonomeren und Pentaerythrittriallylether als Vernetzer verrührt und 30 min mit Stickstoff gespült. Im Stickstoff-Strom wurde auf 80°C unter Rühren erwärmt und nach Erreichen dieser Temperatur während 3 h ein Zulauf aus 80 ml Solvens und 0,3 g Dilauroylperoxid zugegeben. Nach weiteren 4 h wurde abgekühlt, das ausgefallene Produkt abfiltriert, mit Solvens gewaschen und getrocknet. Die Einsatzstoffe und ihre Mengen sind in Tabelle 1 angegeben.

**Tabelle 1**

| Zusammensetzung der Fällungspolymerisate | | | |
|---|---|---|---|
| Bsp. Nr. | Comonomere (Komponente B aus Bsp. Nr.) | Pentaerythrittriallylether | Solvens (vol.-Verhältnis) |
| 9 | 8,0 g 1 | 1,2 g | 1,1,1-Trichlorethan |
| 10 | 8,0 g 2 | 1,2 g | 1,1,1-Trichlorethan |
| 11 | 2,4 g 1 2,4 g Stearylmethacrylat | 1,0 g | 1,1,1-Trichlorethan |
| 12 | 4,0 g 4 | 1,2 g | 1,1,1-Trichlorethan |
| 13 | 8,0 g 4 | 1,2 g | Aceton |
| 14 | 8,0 g 4 | 1,2 g | Essigsäure-isopropylester |
| 15 | 5,0 g 4 | 1,2 g | Essigsäureethyl-ester Cyclohexan (1:1) |
| 16 | 5,0 g 7 | 1,2 g | 1,1,1-Trichlorethan |
| 17 | 5,0 g 4 | 1,2 g | Essigsäureethyl-ester/ Cyclohexan (1:1) |
| 18 | 2,5 g 4 2,5 g Stearylmethacrylat | 0,8 g | Essigsäureethyl ester/ Cyclohexan (1:1) |
| 19 | 5,0 g 6 | 1,2 g | Essigsäureethyl ester/ Cyclohexan (1:1) |
| 20 | 10,0 g 6 | 1,2 g | Cyclohexan |
| 21 | 1,0 g 4 5,0 g Stearylmethacrylat | 1,2 g | Cyclohexan |
| 22 | 10,0 g 5 | 1,2 g | Essigsäure-isobutylester |
| 23 | 5,0 g 8 | 1,2 g | Essigsäureethyl-ester/ Cyclohexan (1:1) |
| 24 | 10,0 g 8 | 1,2 g | Essigsäureethyl-ester/Cyclohexan (1:3) |
| 25 | 4,0 g 4 | 0,6 g | 1,1,1-Trichlorethan |
| 26 | 4,0 g 4 | 0,3 g | 1,1,1-Trichlorethan |

### Vergleichsbeispiel A

Die Reaktion erfolgte analog Beispiel 9 bis 26 mit 5,0 g Stearylmethacrylat als alleinigem Comonomer und 1,2 g Pentaerythrittriallylether in 1,1,1-Trichlorethan.

### Vergleichsbeispiel B

Die Reaktion erfolgte analog Beispiel 9 bis 26 mit 10,0 g Stearylmethacrylat als alleinigem Comonomer und 1,2 g Pentaerythrittriallylether in einem Gemisch aus Essigsäureethylester und Cyclohexan im Vol.-Verhältnis 1 : 1.

### Beispiele 27 bis 36 (Suspensionspolymerisate)

In einem 3 l-Planschliffkolben wurden 1000 ml Cyclohexan und ein Schutzkolloid oder Emulgator vorgelegt. Nach 30 min Begasung mit Stickstoff wurde unter Rühren bei 75°C während 30 min ein Zulauf aus 100 g Wasser, 100 g Acrylsäure, 1 g Kaliumperoxodisulfat sowie Comonomeren und gegebenenfalls Vernetzer zugetropft. Nach weiteren 3 h steigerte man die Temperatur bis zum Sieden und destillierte das Wasser azeotrop ab. Die zurückbleibende Suspension des Polymeren wurde abfiltriert, mit Cyclohexan gewaschen und anschließend im Vakuum getrocknet. Die Einsatzstoffe und ihre Mengen sind in Tabelle 2 angegeben.

**Tabelle 2**

| Zusammensetzung der Suspensionspolymerisate | | | |
|---|---|---|---|
| Bsp. Nr. | Comonomere (Komponente B aus Bsp. Nr.) | Vernetzer | Schutzkolloid/Emulgator |
| 27 | 4,0 g 1 | - | 2 g SMC |
| 28 | 4,0 g 1 | 0,8 g Diallylweinsäurediamid | 3 g SMC |
| 29 | 33,0 g SPMAEDMA 5,3 g 1 | - | 2 g SMC |
| 30 | 4,0 g 1 | - | 2 g Dowfax 2A1 |
| 31 | 2,0 g 1 | - | 3 g Dowfax 2A1 |
| 32 | 4,0 g 6 | 0,1 g Bisacrylamidoessigsäure | 2 g Dowfax 2A1 |
| 33 | 2,0 g 4 | 0,1 g Polyethylen-glykol-200-bis-acrylat | 4 g eines technischen Stearylalkohols mit Ethoxylierungsgrad n=7 |
| 34 | 2,0 g 6 | - | 4 g Dowfax 2A1 |
| 35 | 4,0 g 4 | - | 2 g Dowfax 2A1 |
| 36 | 2,0 g 4 | 0,1 g Methylenbisacrylamid | 2 g Polyvinyl-pyrrolidon |
| SMC = Styrol-Maleinsäure-Copolymer (90:10) | | | |
| SPMAEDMA = 3-Sulfopropylmethacryloyloxyethyldimethylammoniumbetain | | | |
| Dowfax 2A1 = Natriumsalz einer Disulfonsäure eines alkylierten Diphenylethers. | | | |

### Anwendungstechnische Eigenschaften

### Herstellung von Gelen

In einem Becherglas wurden jeweils 1,0 g des Polymerisats aus den Beispielen 9 bis 36 und den Vergleichsbeispielen A und B in 190 ml Wasser dispergiert. Unter Rühren wurden 10 ml einer 10%igen Triethanolaminlösung zugegeben.

Die Viskosität der erhaltenen Gele wurde mit einem Handviskosimeter (Haake VT-02) bestimmt (Ergebnisse siehe Tabelle 3). Durch Ausstreichen auf einer Glasplatte überprüfte man anschließend die glatte Struktur der Gele nach Augenschein.

Die Gele wurden wie angegeben in Wasser und in 1%iger NaCl-Lösung angesetzt. Der Viskositätsvergleich beider Gele belegt die höhere Salzstabilität der erfindungsgemäßen partiell ampholytischen Polymerisate (Ergebnisse siehe Tab.4).

### Herstellung von Emulsionen

In ein Becherglas wurden 0,4 g Polymerisat eingewogen und in 30 ml Paraffinöl dispergiert. Danach wurden 100 ml Wasser und anschließend 4 ml einer 10 %igen Triethanolamin-Lösung unter gutem Rühren zugegeben. Die Emulsion wurde dann mit einem Dispergieraggregat bei 8000 U/min für wenige s homogenisiert. Die Viskosität wurde wie oben bestimmt (Ergebnisse siehe Tabelle 3). Die Langzeitstabilität wurde überprüft, indem die Emulsion in einem 100 ml-Standzylinder auf eventuelle Phasentrennungen nach 14 d kontrolliert wurde.

**Tabelle 3**

| Gel- und Emulsions-Viskositäten | | |
|---|---|---|
| Beispiel Nr. | Gel-Viskosität [Pas] | Emulsions-Viskosität [Pas] |
| Vergl.-Bsp. A | 12 | 9 |
| Vergl.-Bsp. B | 5 | 4,3 |
| 9 | 8,5 | 6,2 |
| 10 | 4,5 | 6,4 |
| 11 | 13 | 13,9 |
| 12 | 20 | 19,8 |
| 13 | 16 | 10,2 |
| 14 | 9,2 | 7,8 |
| 15 | 10,1 | 8,8 |
| 16 | - Quellkörperbildung - | - |
| 17 | 18,0 | 11,1 |
| 18 | 6,3 | 3,9 |
| 19 | 7,5 | 11,0 |
| 20 | 4,1 | 6,4 |
| 21 | 12,5 | 11,7 |
| 22 | 14 | 12 |
| 23 | 9,2 | 13 |
| 24 | 12,1 | 9,6 |
| 25 | 15,2 | 14,1 |
| 26 | 10,8 | 10 |
| 27 | 4,1 | 3,8^{a)} |
| 28 | - Quellkörperbildung - | - |
| 29 | 3,9 | 4,8 |
| 30 | 5,2 | 4,7^{a)} |
| 31 | 3,9 | 4,6 |
| 32 | 11,2 | 9,6 |
| 33 | 8,4 | 6,6 |
| 34 | 4,6 | 5,3 |
| 35 | 7,6 | 8,1 |
| 36 | 10,4 | 12 |

| | | |
|---|---|---|
| ^{a)} geringe Ölausscheidung nach 14 h | | |

**Tabelle 4**

| Viskositätsvergleich der Gele in Wasser und NaCl-Lösung | |
|---|---|
| Beispiel Nr. | Viskosität in Wasser/Viskosität in 1 %iger NaCl-Lösung |
| Vergl.-Bsp. A | 200 |
| Vergl.-Bsp. B | 180 |
| 10 | 30 |
| 11 | 70 |
| 15 | 45 |
| 20 | 25 |
| 23 | 40 |
| 25 | 60 |
| 29 | 50 |

Durch Ausstreichen der Emulsion aus Tabelle 3 auf eine Glasplatte mit einem flachen Spatel und mikroskopische Betrachtung des dünnen Films konnte die Dispergierfähigkeit der Polymerisate kontrolliert werden. Es ergaben sich folgende mittlere Teilchengrößen:

| | |
|---|---|
| Vergleichsbeispiel A | 30 µm |
| Beispiel 10 | 8,5 µm |
| Beispiel 13 | 20 µm |
| Beispiel 18 | 15 µm |
| Beispiel 19 | 6 µm |

Die kleinere Teilchengröße der mit den erfindungsgemäßen Polymerisaten erhaltenen Emulsionen war ein Indiz für die verbesserte Emulsionsstabilität dieser Phasen.

## Patentansprüche

1. Copolymerisat, erhältlich durch radikalisch initiierte Polymerisation von
A) 50 bis 99,99 Gew.-% einer olefinisch ungesättigten C₃- bis C₅-Monocarbonsäure, einer olefinisch ungesättigten C₄- bis C₈-Dicarbonsäure oder ihres Anhydrids oder einer Mischung solcher Carbonsäuren oder Carbonsäureanhydride mit
B) 0,01 bis 50 Gew.-% einer olefinisch ungesättigten quartären Ammoniumverbindung der allgemeinen Formel oder II 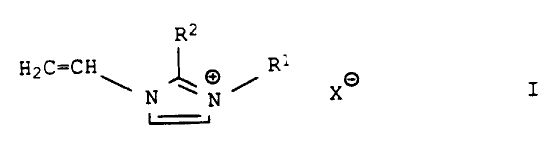 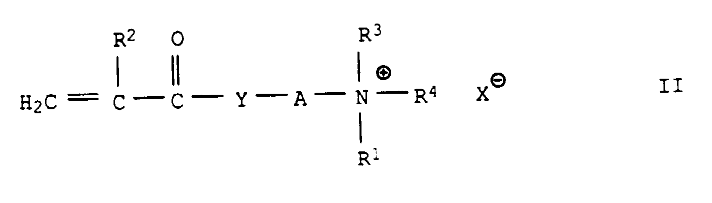 in den
R¹ C₆- bis C₂₀-Alkyl, C₆- bis C₂₀-Alkenyl, C₅- bis C₈-Cycloalkyl, Phenyl, Phenyl(C₁- bis C₁₂-alkyl) oder (C₁- bis C₁₂-Alkyl)phenyl bedeutet,
R² Wasserstoff, Methyl oder Phenyl bezeichnet,
R³ und R⁴ für Wasserstoff oder C₁- bis C₄-Alkyl stehen,
X Halogen, C₁- bis C₄-Alkylsulfat oder C₁- bis C₄-Alkylsulfonat bedeutet, wobei eine solche C₁- bis C₄-Alkylsulfonat-Gruppe auch als Rest R³ oder R⁴ unter Ausbildung einer Betainstruktur auftreten kann,
Y für O oder NH steht und
A C₁- bis C₆-Alkylen bezeichnet,
oder einer Mischung solcher Ammoniumverbindungen,
C) 0 bis 49,99 Gew.-% eines Acrylats oder Methacrylats der allgemeinen Formel III 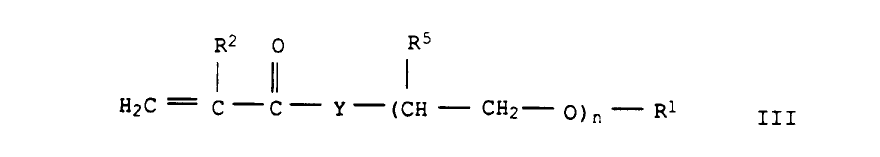 in der R¹, R² und Y die oben genannten Bedeutungen haben, R⁵ Wasserstoff, Methyl oder Ethyl bezeichnet und n für eine Zahl von 0 bis 25 steht,
D) 0 bis 49,99 Gew.-% weiterer copolymerisierbarer Monomerer und
E) 0 bis 5 Gew.-% einer oder mehrerer Verbindungen mit mindestens zwei olefinisch ungesättigten Gruppen im Molekül als Vernetzer.

2. Copolymerisat nach Anspruch 1, erhältlich durch radikalisch initiierte Polymerisation von
A) 70 bis 99,85 Gew.-% der Carbonsäure-Komponente A,
B) 0,1 bis 29,95 Gew.-% der quartären Ammoniumverbindung I oder II,
C) 0 bis 29,85 Gew.-% des Acrylats oder Methacrylats III,
D) 0 bis 29,85 Gew.-% weiterer copolymerisierbarer Monomerer und
E) 0,05 bis 2 Gew.-% der Vernetzer-Komponente E.

3. Copolymerisat nach Anspruch 1 oder 2, bei dessen Herstellung als Komponente A Acrylsäure, Methacrylsäure oder Maleinsäureanhydrid verwendet wurden.

4. Copolymerisat nach den Ansprüchen 1 bis 3, bei dessen Herstellung als Komponente B quartäre Ammoniumverbindungen I oder II eingesetzt wurden, bei denen
R¹ C₁₂- bis C₁₈-Alkyl, C₁₂- bis C₁₈-Alkenyl oder Benzyl bedeutet,
R² Wasserstoff, Methyl oder Phenyl bezeichnet,
R³ und R⁴ für C₁- bis C₄-Alkyl stehen,
X Chlor, Brom, Methylsulfat, Ethylsulfat, Methylsulfonat, Ethylsulfonat oder eine C₁- bis C₃-Alkyl-sulfonat-Gruppe, welche als Rest R³ oder R⁴ unter Ausbildung einer Betainstruktur auftritt, bedeutet,
Y für O oder NH steht und
A C₂- bis C₄-Alkylen bezeichnet,

5. Copolymerisat nach den Ansprüchen 1 bis 4, bei desser Herstellung als Komponente E Pentaerythrittriallylether, Oleylmethacrylat, Diallylweinsäurediamid, Bisacrylamidoessigsäure, Methylenbisacrylamid oder ein Polyethylenglycoldi(meth)acrylat verwendet wurden.

6. Verwendung von Copolymerisaten gemäß den Ansprüchen 1 bis 5 als Verdickungs- oder Dispergiermittel zur Verdikkung wäßriger Systeme.

7. Kosmetische Zubereitungen, enthaltend Copolymerisate gemäß den Ansprüchen 1 bis 5 als Verdickungs- oder Dispergiermittel in den hierfür üblichen Mengen.

## Claims

1. A copolymer obtainable by free-radical polymerization of
A) 50-99.99% by weight of an olefinically unsaturated C₃-C₅ monocarboxylic acid, of an olefinically unsaturated C₄-C₈ dicarboxylic acid or the anhydride thereof or a mixture of such carboxylic acids or anhydrides with
B) 0.01-50% by weight of an olefinically unsaturated quaternary ammonium compound of the formula I or II 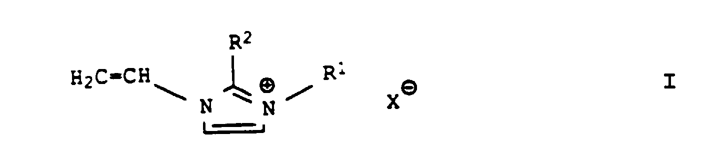 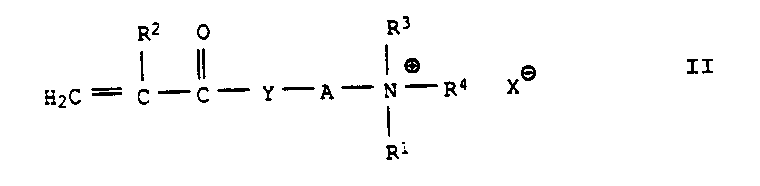 where
R¹ is C₆-C₂₀-alkyl, C₆-C₂₀-alkenyl, C₅-C₈-cycloalkyl, phenyl, phenyl(C₁-C₁₂-alkyl) or (C₁-C₁₂-alkyl)phenyl,
R² is hydrogen, methyl or phenyl,
R³ and R⁴ are each hydrogen or C₁-C₄-alkyl,
X is halogen, C₁-C₄-alkyl sulfate or C₁-C₄-alkylsulfonate, it also being possible for the latter to occur as R³ or R⁴ with the formation of a betaine structure,
Y is O or NH, and
A is C₁-C₆-alkylene,
or a mixture of such ammonium compounds,
C) 0 to 49.99% by weight of an acrylate or methacrylate of the formula III 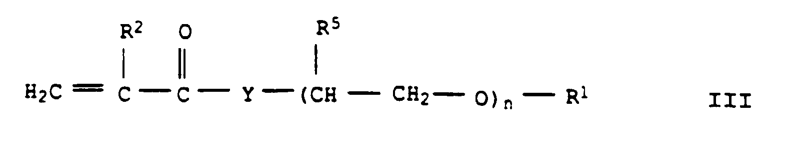 where R¹, R² and Y have the abovementioned meanings, R⁵ is hydrogen, methyl or ethyl, and n is a number from 0 to 25,
D) 0-49.99% by weight of other copolymerizable monomers and
E) 0-5% by weight of one or more compounds with at least two olefinically unsaturated groups in the molecule as crosslinker.

2. A copolymer as claimed in claim 1, obtainable by free-radical polymerization of
A) 70-99.85% by weight of the carboxylic acid component A,
B) 0.1-29.95% by weight of the quaternary ammonium compound I or II,
C) 0-29.85% by weight of the acrylate or methacrylate III,
D) 0-29.85% by weight of other copolymerizable monomers and
E) 0.05-2% by weight of the crosslinker component E.

3. A copolymer as claimed in claim 1 or 2, in whose preparation acrylic acid, methacrylic acid or maleic anhydride has been used as component A.

4. A copolymer as claimed in claims 1 to 3, in whose preparation quaternary ammonium compounds I or II where
R¹ is C₁₂-C₁₈-alkyl, C₁₂-C₁₈-alkenyl or benzyl,
R² is hydrogen, methyl or phenyl,
R³ and R⁴ are each C₁-C₄-alkyl,
X is chlorine, bromine, methyl sulfate, ethyl sulfate, methylsulfonate, ethylsulfonate or C₁-C₃-alkylsulfonate, which occurs as R³ or R⁴ with formation of a betaine structure,
Y is O or NH, and
A is C₂-C₄-alkylene, have been used as component B.

5. A copolymer as claimed in claims 1 to 4, in whose preparation pentaerythritol triallyl ether, oleyl methacrylate, diallyltartaramide, bisacrylamidoacetic acid, methylenebisacrylamide or a polyethylene glycol di(meth)acrylate has been used as component E.

6. The use of copolymers as claimed in claims 1 to 5 as thickeners or dispersants for thickening aqueous systems.

7. A cosmetic composition containing copolymers as claimed in claims 1 to 5 as thickeners or dispersants in the amounts customary for this purpose.

## Revendications

1. Copolymère obtenu par polymérisation radicalaire de
**A)** 50 à 99,99 % en poids d'un acide monocarboxylique à insaturation oléfinique en C3-C5, d'un acide dicarboxylique à insaturation oléfinique en C4-C8 ou de son anhydride ou d'un mélange de ces acides carboxyliques ou anhydrides carboxyliques, avec
**B)** 0,01 à 50 % en poids d'un composé d'ammonium quaternaire à insaturation oléfinique de formule générale I ou II 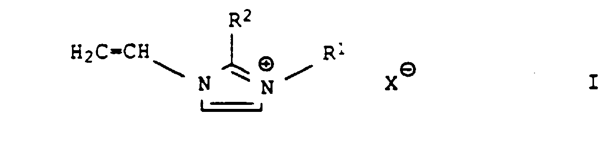 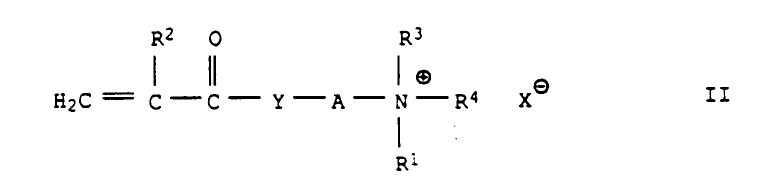 dans lesquelles
R¹ représente un groupe alkyle en C6-C20, alcényle en C6-C20, cycloalkyle en C5-C8, phényle, phényl-alkyle en C1-C12 ou (alkyle en C1-C12)phényle,
R² représente l'hydrogène, un groupe méthyle ou phényle,
R³ et R⁴ représentent l'hydrogène ou des groupes alkyle en C1-C4,
X représente un halogène, un groupe alkylsulfate en C1-C4 ou alkylsulfonate en C1-C4, un tel groupe alkylsulfonate en C1-C4 pouvant également exister en tant que substituant R³ ou R⁴ avec formation d'une structure de bétaïne,
Y représente O ou NH et
A représente un groupe alkylène en C1-C6, ou d'un mélange de tels composés d'ammonium,
**C)** 0 à 49,99 % en poids d'un acrylate ou méthacrylate de formule générale III 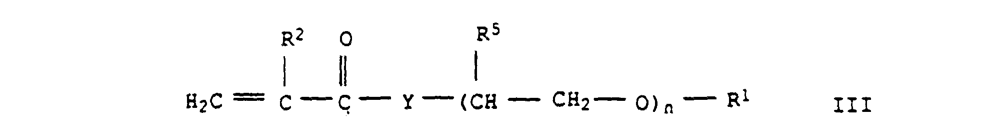 dans laquelle R¹, R² et Y ont les significations indiquées ci-dessus, R⁵ représente l'hydrogène, un groupe méthyle ou éthyle et n est un nombre allant de 0 à 25,
**D)** 0 à 49,99 % en poids d'autres monomères copolymérisables et
**E)** 0 à 5 % en poids d'un ou plusieurs composés contenant dans la molécule au moins deux groupes à insaturation oléfinique et qui servent d'agents réticulants.

2. Copolymère selon la revendication 1, obtenu par polymérisation radicalaire de
**A)** 70 à 99,85 % en poids du composant acide carboxylique A,
**B)** 0,1 à 29,95 % en poids du composé d'ammonium quaternaire I ou II,
**C)** 0 à 29,85 % en poids de l'acrylate ou méthacrylate III,
**D)** 0 à 29,85 % en poids d'autres monomères copolymérisables et
**E)** 0,05 à 2 % en poids du composant réticulant E.

3. Copolymère selon l'une des revendications 1 ou 2, à la préparation duquel on a utilisé, en tant que composant A, l'acide acrylique, l'acide méthacrylique ou l'anhydride maléique.

4. Copolymère selon les revendications 1 à 3, à la préparation duquel on a utilisé, en tant que composant B, des composés d'ammonium quaternaire I ou II pour lesquels
R¹ représente un groupe alkyle en C12-C18, alcényle en C12-C18 ou benzyle,
R² représente l'hydrogène, un groupe méthyle ou phényle,
R³ et R⁴ représentent des groupes alkyle en C1-C4,
X représente le chlore, le brome, un groupe méthylsulfate, éthylsulfate, méthylsulfonate, éthylsulfonate ou un groupe alkylsulfonate en C1-C3 existant en tant que substituant R³ ou R⁴, avec formation d'une structure de bétaïne,
Y représente O ou NH et
A représente un groupe alkylène en C2-C4.

5. Copolymère selon l'une des revendications 1 à 4 à la préparation duquel on a utilisé, en tant que composant E, l'éther triallylique du pentaérythritol, le méthacrylate d'oléyle, le diamide diallyltartrique, l'acide bis-acrylamidoacétique, le méthylène-bis-acylamide ou un di(méth)acrylate de polyéthylèneglycol.

6. Utilisation des copolymères selon les revendications 1 à 5 en tant qu'agents épaississants ou dispersants pour l'épaississement de systèmes aqueux.

7. Compositions cosmétiques contenant des copolymères selon les revendications 1 à 5 en tant qu'agents épaississants ou dispersants aux proportions habituelles à cet effet.
